# EUROPEAN PATENT APPLICATION

(11) **EP 4 574 017 A1**
(43) Date of publication of application: **25.06.2025**
(21) Application number: 23854480.3
(22) Date of filing: 17.08.2023
(51) Int. Cl.: A61B 3/107, A61B 3/14, A61B 3/00

(54) **QUANTITATIVE EVALUATION METHOD FOR CONJUNCTIVAL CONGESTION, APPARATUS, AND STORAGE MEDIUM**

(30) Priority: 18.08.2022 CN 202210992159
(71) Applicant: Shanghai Institute Of Endocrine And Metabolic Diseases, Shanghai 200020 (CN); Shangai Baiyi Healthcare Technology Co, Ltd, Shanghai 201203 (CN)
(72) Inventor: WANG, Weiqing, Shanghai 200020 (CN); NING, Guang, Shanghai 200020 (CN); SHEN, Liyun, Shanghai 200020 (CN); TIAN, Chaonan, Shanghai 201203 (CN); DU, Dong, Shanghai 201203 (CN)
(74) Representative: Elzaburu S.L.P.
(86) International application number: PCT/CN2023/113490
(87) International publication number: WO 2024/037581

(57) **Abstract**

The application discloses a method, a device and a storage medium for quantitatively evaluating conjunctival congestion. The method for quantitatively evaluating conjunctival congestion includes the following steps: Step S1: obtaining a video of ocular movement from the inside out or from the outside in; Step S2: segmenting an eye image in the video to obtain an eye conjunctiva image, and extracting a red channel value and a blue channel value of each pixel from the eye conjunctiva image; Step S3: determining the percentage of conjunctival congestion by the ratio of the red channel value to the blue channel value of each pixel. The precise value of conjunctival congestion can be obtained by the quantitative evaluation method.

## Description

### TECHNICAL FIELD

The invention relates to the technical field of eye detection, in particular to a method, a device and a storage medium for quantitatively evaluating conjunctival congestion.

### BACKGROUND

The degree of conjunctival congestion is an important reference index for doctors to evaluate the ocular inflammation of patients. The clinical practice, doctors require that the degree of conjunctival congestion (light, medium or heavy) be judged manually, within 1 m of the patient. Because of the subjectivity of different doctors, there is no uniform standard for the judgment of light, medium and heavy conjunctival congestion, which can result in misdiagnosis and missed diagnosis.

Aiming at the problem with accuracy of manual measurement as affected by human factors, the prior art proposed a shape-dependent image recognition technology scheme. As described in the Chinese patent CN111212594A (Application No.: CN201880066273.2, published on May 29, 2020). Capture images including the eyes through a camera, identify one or more blood vessels in the image, and the degree of conjunctival congestion is determined based on the size of the one or more blood vessels identified. In addition, in the prior art, there is another method to check the degree of conjunctival congestion by using the proportion of the red pixel value of the eye. Specifically, this method obtains high-resolution eye images. Then converts the eye images from RGB to gray, uses Gaussian filtering to identify the regions with significant differences in horizontal and vertical gradients. And uses a threshold value to divide the bloodshot and non-bloodshot on the filtered heat map.

However, eye congestion is mostly conjunctival congestion, and the congestion in this area is mostly diffuse. Therefore, shape-dependent recognition method used in the prior art has the following disadvantages: 1. The depth of blood vessel color can easily affect the accuracy of extracting red pixel values, leading to inaccurate recognition; 2. Due to the characteristics of the structure of the human eyeball, there will be shadows at the intersection of the eyelid and the eyeball, which can affect the accuracy; 3, Because the eyeball is spherical, use of a single light beam to irradiate the eyeball results in uneven light.

### SUMMARY

**In** order to overcome the low accuracy of the prior art in measuring the degree of conjunctival congestion, the invention discloses a method for quantitatively evaluating conjunctival congestion. **In** order to reach the above purpose, the invention adopts the following technical scheme:
**In** one aspect, the present invention discloses a method for quantitatively evaluating conjunctival congestion, including the following steps:
Step S1: obtaining a video of eyeball movement from the inside out or from the outside in;
Step S2: segmenting an eye image in the video to obtain eye conjunctiva image, and extracting a red channel value and a blue channel value of each pixel from the eye conjunctiva image; and
Step S3: determining a percentage of conjunctival congestion from a ratio of the red channel value to the blue channel value of each pixel.

Furthermore, a second eye position image of the eyeball respectively moving inward and outward to a limit position is obtained respectively in the step S2, and the eye conjunctival image is obtained by combining the second eye position image of the eyeball moving inward to limit position and the second eye position image of the eyeball moving outward to limit position.

Furthermore, in step S3, identify pixels in which the ratio of the red channel value to the blue channel value exceeds a threshold as red bloodshot, and the percentage of conjunctival congestion is the percentage of the area of the red bloodshot pixels in the conjunctival area of the eye conjunctival image.

Furthermore, the threshold is a fixed value set in advance, or a manually set value.

In another aspect, the invention discloses a device for quantitatively evaluating conjunctival congestion, comprising:
A video acquisition module, configured to respond to a video acquisition instruction and obtain a video of eyeball movement from the inside out or from the outside in;
A feature extraction module, configured to segment the eye image to obtain an eye conjunctiva image, and extract a red channel value and a blue channel value of each pixel in the eye conjunctiva image; and
A calculation module, configured to calculate the ratio of the red channel value to the blue channel value of each pixel to determine the percentage of conjunctival congestion.

Furthermore, the feature extraction module includes an image processing submodule, configured to extract a second eye position image of the eyeball moving inward and outward to a limit position from the video.

In another aspect, the invention discloses a computer-readable storage medium, wherein at least one program code is stored in the computer-readable storage medium, and the at least one program code is loaded by a processor and performs a method for quantitatively evaluating conjunctival congestion as described in the first aspect.

Compared with the prior art, the invention has at least the following beneficial effects:
In the invention, the eye conjunctiva image is synthesized by a second eye position image of the eyeball moving inward and outward to the limit position, and then the red channel value and the blue channel value are extracted from each pixel of the image. Since the light reflected by the bloodshot area is mainly red, and the sclera itself has a high reflectivity for both red and blue lights, the red channel value is significantly higher in the bloodshot area than the blue channel value. Where there is no bloodshot, the red channel value and the blue channel value are equivalent, and the congestion point can be accurately determined by the ratio of the red channel value and the blue channel value of each pixel, so as to determine the percentage of conjunctival congestion.

### DESCRIPTION OF THE DRAWINGS

FIG. 1 is a flow chart of a method for quantitatively evaluating conjunctival congestion of the present invention;
FIG. 2 is a schematic block diagram for the quantitative evaluation of conjunctival congestion;
FIG. 3 is a procedure diagram for the quantitative evaluation of conjunctival congestion of the present invention;
FIG. 4 is a structural diagram of a device for quantitatively evaluating of conjunctival congestion.

### EXAMPLES

The specific embodiments of the invention are further described in detail in combination with the attached drawings and embodiments. The following embodiments are used to illustrate the invention, but not to limit the scope of the invention.

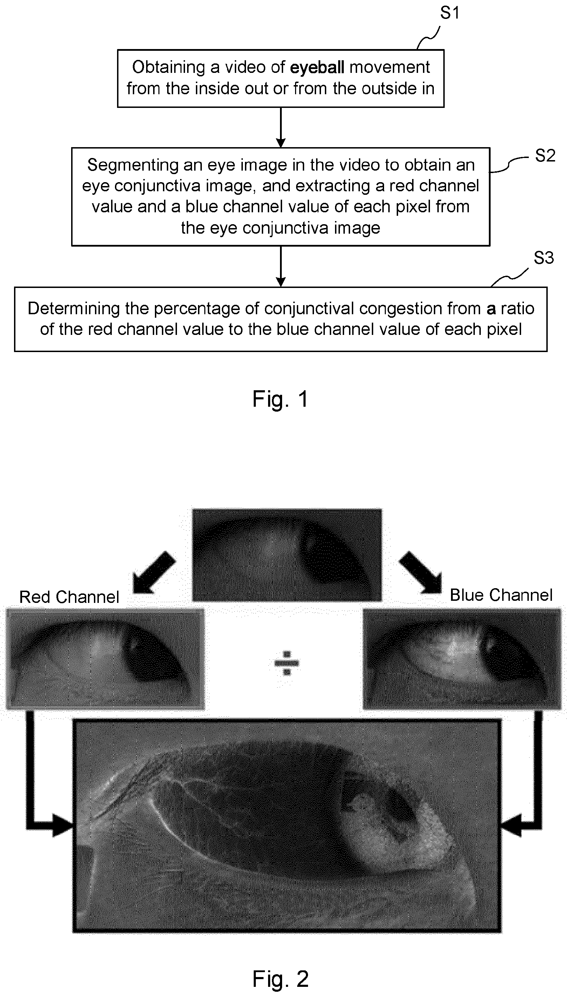

As shown in Figs. 1-3, a method for quantitatively evaluating conjunctival congestion includes the following steps:
Step S1: obtaining a video of eyeball movement from the inside out or from the outside in.

Specifically, in step S1, the eye evaluation or measurement device can be used to fix the user's eyes and head, and the camera set in front of the user's eyes can capture video of the eyeball moving horizontally from inside to outside or from outside to inside, and an indicator light can be used to guide the user's eyeball movement.

Video of the desired eyeball movements can also be obtained through other eye devices or video capture devices.

Step S2: segmenting an eye image in the video to obtain an eye conjunctiva image, and extracting a red channel value and a blue channel value of each pixel from the eye conjunctiva image.

Specifically, in step S2, segmentation of an eye image from the video can obtain an eye conjunctiva image with the eyeball removed. Specifically, second eye position images with the eyeball moving inward and outward to respective limit positions can be extracted from the video. Then, the extracted the second eye position image with the eyeball moving inward to the limit position and the extracted the second eye position image with the eyeball moving outward to the limit position are superimposed to obtain the eye conjunctiva image without the influence of the eyeball image. Then, the red channel value and blue channel value of each pixel are extracted from the eye conjunctiva image.

It should be noted that the eye position refers to the position of the eyeball during the eye examination, which is divided into a first eye position, a second eye position and a third eye position. The first eye position refers to the eye gaze position when the two eyes look straight ahead towards an infinite distance in a horizontal plane, the second eye position refers to the eye position when the eyeball moves up, down, in and out, and the third eye position refers to the eye position when the eyeball moves inwardly up, inwardly down, outwardly up and outwardly down, that is, the eye position when the eyeball moves up, under the nose, supratemporally and subtemporally. Accordingly, the first, second and third eye position images refer to the images taken of the eye at each eye gaze position, respectively.

Step S3: determining a percentage of conjunctival congestion from a ratio of the red channel value to the blue channel value of each pixel.

In Step S3, identify pixels in which the ratio of the red channel value to the blue channel value that exceeds a threshold as red bloodshot, and the percentage of conjunctival congestion is the percentage of the area of the red bloodshot pixels in the conjunctival area of the eye conjunctival image. Since the light reflected by the bloodshot area is mainly red light, and the sclera itself has a high reflectivity for both red and blue lights, the red channel value is significantly higher in the bloodshot area than the blue channel value, while the red channel value and the blue channel value are equivalent in the absence of a bloodshot area. Therefore, the percentage of conjunctival congestion is determined by the ratio of the red channel value to the blue channel value in each pixel. Specifically, the threshold is a fixed value set in advance. Alternatively, the threshold is a manually set value. For example, a user can enter a threshold value of 3 in the computer in advance to set pixels having a ratio of the red channel value to the blue channel value exceeding 3 as red bloodshot. If the RGB value of a pixel is RGB (250,245,240), then the red channel value and the blue channel value are equivalent, and the ratio of the two is close to 1, so the pixel is not red bloodshot, but the white of the sclera itself. If the RGB value of a pixel is RGB (250,20,15), then the ratio of the red channel value to the blue channel value exceeds the threshold value 3, so the pixel is determined to be red bloodshot. In addition to the threshold entered in advance, the threshold can also be a value manually set by a technician such as a doctor or operator based on his/her experience.

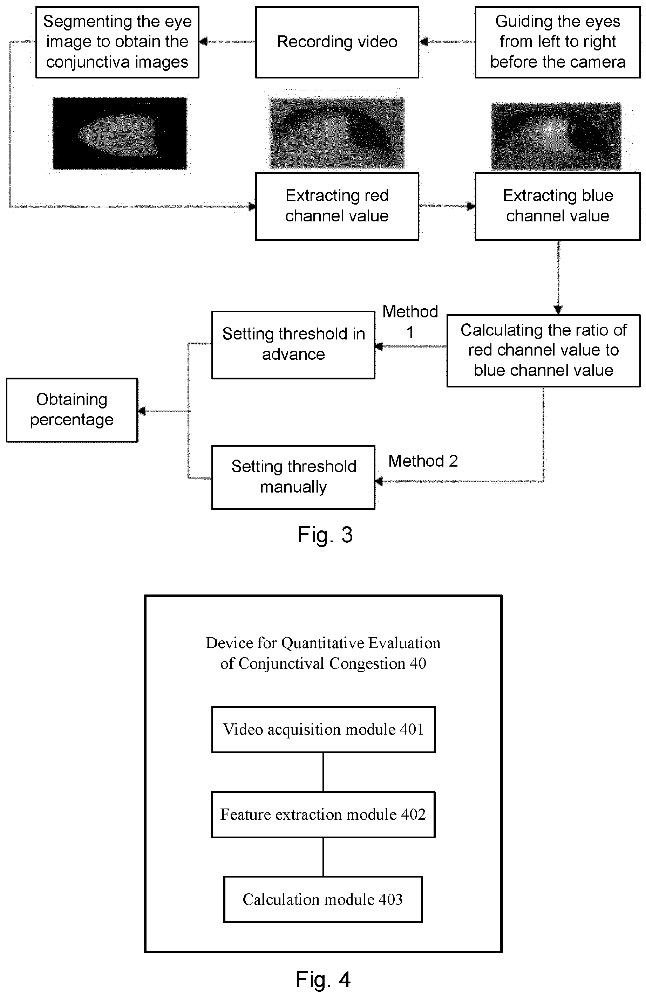

Fig. 3 more specifically shows an exemplary step diagram of the method of quantitatively evaluating conjunctival congestion. First, the user controls the left and right movement of the eyeball in front of the camera, and the video is recorded. Next, the eye image is segmented. During the segmentation of the eye image, a second eye position image of the eyeball moving to the respective limit positions is obtained. The image of the eye conjunctiva is obtained by superimposing the second eye position image with the eyeball moving to the inward limit position and the second eye position image with the eyeball moving to the outward limit position. Then, the red channel values and blue channel values of each pixel in the eye conjunctiva image are extracted, and the ratio of the red and blue channel pixel values of each pixel is further obtained.

And according to the pre-set threshold or manually set threshold to determine whether each pixel is red bloodshot, the percentage of red bloodshot is obtained.

Fig. 4 is a structural diagram of a device 40 for the quantitative evaluation of conjunctival congestion, provided by an embodiment of the invention. The device comprises:
A video acquisition module 401, configured to respond to a video acquisition instruction and obtain a video of eyeball movement from the inside out or from the outside in. In one possible implementation, the video acquisition module can control the start of the camera or other filming equipment when receiving the acquisition instruction, guide the user's eye movement by controlling a voice device or a light device, and obtain the eyeball movement video.

A feature extraction module 402, configured to segment the eye image to obtain the eye conjunctiva image, and extract the red channel value and blue channel value of each pixel from the eye conjunctiva image; and
A calculation module 403, configured to calculate the ratio of the red channel value to the blue channel value of each pixel to determine the percentage of conjunctival congestion. Furthermore, the feature extraction module may include an image processing submodule, configured to extract a second eye position image of the eyeball moving inward and outward to the limit positions from the video.

In an exemplary embodiment, the invention discloses a computer-readable storage medium, wherein at least one program code is stored in the computer-readable storage medium, and the at least one program code is loaded by a processor and performs a method for quantitatively evaluating conjunctival hyperemia as described above. For example, the computer-readable storage medium can be Read-Only Memory (ROM), Random Access Memory (RAM), Compact Disc Read-Only Memory (CDROM), magnetic tape, floppy disk and optical data storage devices. A person of ordinary skill in the art may understand that all or part of the steps to implement the above embodiments may be performed by hardware or by hardware associated with at least one program code, which may be stored in a computer-readable storage medium, such as read-only memory, disk or optical disc.

The above are only preferred embodiments of this application and are not intended to limit the invention, and any modification, equivalent replacement, improvement, etc. made within the spirit and principles of the invention shall be included in the scope of protection of the invention.

## Claims

1. A method for quantitatively evaluating conjunctival congestion, **characterized in that** it includes the following steps:
Step S1: obtaining a video of eyeball movement from the inside out or from the outside in;
Step S2: segmenting an eye image in the video to obtain an eye conjunctiva image, and extracting a red channel value and a blue channel value of each pixel from the eye conjunctiva image; and
Step S3: determining a percentage of conjunctival congestion from a ratio of the red channel value to the blue channel value of each pixel.

2. The method for quantitatively evaluating conjunctival congestion in claim 1, **characterized in that** a second eye position image of the eyeball respectively moving inward and outward to a limit position is obtained in step S2, and the eye conjunctival image is obtained by combining the second eye position image of the eyeball moving inward to a limit position and the second eye position image of the eyeball moving outward to the limit position.

3. The method for quantitatively evaluating conjunctival congestion in claim 1, **characterized in that**: in step S3, identify pixels in which the ratio of the red channel value to the blue channel value exceeds a threshold as red bloodshot, and the percentage of conjunctival congestion is the percentage of the area of the red bloodshot pixels in a conjunctival area of the eye conjunctival image.

4. The method for quantitatively evaluating conjunctival congestion described in claim 3, **characterized in that** the threshold is a fixed value set in advance or a manually set value.

5. A device for quantitatively evaluating conjunctival congestion, **characterized in that** the device comprises:
a video acquisition module, configured to respond to a video acquisition instruction and obtain a video of eyeball movement from the inside out or from the outside in;
a feature extraction module, configured to segment the eye image to obtain an eye conjunctiva image, and extract a red channel value and a blue channel value of each pixel from the eye conjunctiva image; and
a calculation module, configured to calculate a ratio of the red channel value to the blue channel value of each pixel to determine a percentage of conjunctival congestion.

6. The device for quantitatively evaluating conjunctival congestion in claim 5, **characterized in that** the feature extraction module includes:
an image processing submodule, configured to extract a second eye position image of the eyeball moving inward and outward to a limit position from the video, and to obtain the eye conjunctiva image by combining the second eye position image of the eyeball moving inward to the limit position and the second eye position image of the eyeball moving outward to the limit position.

7. A computer-readable storage medium, **characterized in that** at least one program code is stored in the computer-readable storage medium, and the at least one program code is loaded by a processor and performs the method for quantitatively evaluating conjunctival congestion as described in any of Claims 1-4.
